(19) Europäisches Patentamt
European Patent Office
Office européen des brevets

(11) Veröffentlichungsnummer: **0 513 619 A2**

(12) # EUROPÄISCHE PATENTANMELDUNG

(21) Anmeldenummer: **92107528.9**

(22) Anmeldetag: **04.05.92**

(51) Int. Cl.5: **C07C 39/00**, C07C 321/00, C08K 5/00, //(C08K5/00,5:13, 5:37)

(30) Priorität: **14.05.91 DE 4115621**

(43) Veröffentlichungstag der Anmeldung:
**19.11.92 Patentblatt 92/47**

(84) Benannte Vertragsstaaten:
**DE FR GB IT**

(71) Anmelder: **BAYER AG**

**W-5090 Leverkusen 1 Bayerwerk(DE)**

(72) Erfinder: **Podder, Chiraranjan, Dr.**
**Bitterstrasse 20**
**W-5000 Köln-Worringen(DE)**
Erfinder: **Schlesmann, Harro, Dr.**
**Eifgenstrasse 24**
**W-5068 Odenthal(DE)**
Erfinder: **Eichenauer, Herbert, Dr.**
**Gustav-Heinemann-Strasse 3**
**W-4047 Dormagen 1(DE)**

(54) **Als Stabilisatorsysteme für Polymere geeignete Mischungen.**

(57) Mischungen aus Thioenolethern und phenolischen Antioxidantien wirken stabilisierend auf sauerstoffempfindliche Polymere, insbesondere kautschukmodifizierte Polymere.

EP 0 513 619 A2

Gegenstand der Erfindung sind synergistisch wirkende Mischungen aus phenolischen Antioxidantien und Thioenolethern, die stabilisierend auf sauerstoffempfindliche Polymere, insbesondere kautschukmodifizierte Polymere wirken.

Synthetische Polymere, insbesondere solche mit ungesättigten Bindungen in der Molekülkette, unterliegen unter der Einwirkung von Sauerstoff, Hitze oder Licht Abbaureaktionen, die zu einer Verschlechterung der Polymereigenschaften und zu Problemen beim praktischen Einsatz der aus den Polymeren hergestellten technischen Teile führen.

Zur Verhinderung derartiger Abbaueffekte wurden bereits zahlreiche Stabilisatormischungen für Polymere beschrieben, u.a. auch Kombinationen aus sterisch gehinderten Phenolen und schwefelhaltigen Synergisten. Diesbezüglich kann als Stand der Technik die US-PS 4 321 191 sowie die dort zitierte Literatur genannt werden.

Es wurde nun gefunden, daß Mischungen aus phenolischen Antioxidantien und speziell aufgebauten Thioenolethern eine synergistische Wirkung bezüglich der Stabilisierung von Polymeren gegen die oben erwähnten Abbaureaktionen besitzen. Darüber hinaus können die phenolischen Antioxidantien im Gemisch mit den Thioenolethern ohne Probleme in die zu stabilisierenden Polymere eingearbeitet werden.

Gegenstand der Erfindung sind daher Mischungen aus

A) 1 bis 99 Gew.-Teilen, bevorzugt 10 bis 90 Gew.-Teilen und besonders bevorzugt 20 bis 80 Gew.-Teilen eines Thioenolethers der Formel

$$R^1 = CH\text{-}S\text{-}R^2 ,$$

worin

- $R^1$ einen geradkettigen oder verzweigten Alkenylrest mit 3 bis 18 C-Atomen oder einen substituierten oder unsubstituierten Cycloalkenylrest mit 5 bis 8 C-Atomen bedeutet und
- $R^2$ für einen geradkettigen oder verzweigten Alkylrest mit 3 bis 18 C-Atomen, einen Arylrest mit 6 bis 10 C-Atomen oder einen Aralkylrest mit 7 bis 12 C-Atomen steht, wobei der Alkylrest noch zusätzlich in der Kette durch ein Heteroatom oder mehrere Heteroatome unterbrochen sein kann,

und

B) 99 bis 1 Gew.-Teilen, bevorzugt 90 bis 10 Gew.-Teilen und besonders bevorzugt 80 bis 20 Gew.-Teilen eines phenolischen Antioxidans.

Als geradkettige oder verzweigte Alkenylreste ($R^1$) kommen solche mit 3 bis 18 C-Atomen, bevorzugt 4 bis 8 C-Atomen, in Frage, wie n-Butenyl-, iso-Butenyl-, n-Pentenyl-, iso-Pentenyl-, n-Hexenyl-, n-Heptenyl- und n-Octenylreste, bevorzugt iso-Butenyl- und n-Hexenylreste; als substituierte oder unsubstituierte Cycloalkenylreste solche mit 5 bis 8 C-Atomen, bevorzugt 5 bis 6 C-Atomen, wie Cyclopentenyl-, methylsubstituierte Cyclopentenyl-, Cyclohexenyl- und methylsubstituierte Cyclohexenylreste, bevorzugt Cyclohexenyl- und methylsubstituierte Cyclohexenylreste.

Als geradkettige oder verzweigte Alkylreste ($R^2$) mit 3 bis 18 C-Atomen, bevorzugt 8 bis 18 C-Atomen, die durch ein Heteroatom oder mehrere Heteroatome, wie O, S, N, unterbrochen sein können, kommen beispielsweise in Frage: der n-Octyl-, n-Decyl-, n-Dodecyl-, tert.-Dodecyl- und n-Octadecylrest, bevorzugt der n-Dodecyl-, tert.-Dodecyl- und n-Octadecylrest; als substituierte und unsubstituierte Arylreste mit 6 bis 10 C-Atomen, bevorzugt mit 6 C-Atomen kommen in Frage: der Phenyl-, o-, m- oder p-Methylphenyl-, Naphthyl- und methylsubstituierte Naphthylrest, bevorzugt der Phenyl- und methylsubstituierte Phenylrest; als Aralkylreste mit 7 bis 12 C-Atomen, bevorzugt 7 bis 8 C-Atomen, kommen in Frage: der Benzyl- und Phenethylrest, bevorzugt der Benzylrest.

Die Herstellung der erfindungsgemäßen Thioenolether kann in üblicher Weise erfolgen, z.B. durch Umsetzung geeigneter Aldehyde mit Mercaptanen unter Säurekatalyse zu den entsprechenden Thioacetalen. In einem zweiten Reaktionsschritt werden die primär entstandenen Verbindungen durch thermische Behandlung unter Abspaltung von Mercaptan in die entsprechenden Thioenolether überführt. Thioenolether entstehen auch direkt aus Mercaptan und Aldehyd, wenn man im Molverhältnis 1:1 arbeitet.

Als Beispiele für geeignete Thioenolether seien genannt:
2-Methyl-1-propenyl-tert.-dodecylthioether,
1-Hexenyl-n-dodecylthioether,
Cyclohexylidenmethyl-n-dodecylthioether,
3-Cyclohexen-(1)-ylidenmethyl-n-dodecylthioether,
3-Cyclohexen-(1)-ylidenmethyl-n-octadecylthioether,
3-Cyclohexen-(1)-ylidenmethyl-tert.-dodecylthioether,
3-Cyclohexen-(1)-ylidenmethyl-n-octylthioether,

3-Cyclohexen-(1)-ylidenmethyl-cyclohexylthioether,
3-Methyl-(3)-cyclohexen-(1)-ylidenmethyl-n-dodecylthioether,
3-Cyclohexen-(1)-ylidenmethyl-p-tolylthioether und
3-Cyclohexen-(1)-ylidenmethyl-benzylthioether.

Bevorzugt sind 3-Cyclohexen-(1)-ylidenmethyl-n-dodecylthioether und 1-Hexenyl-n-dodecylthioether.

Die Thioenolether können allein oder im Gemisch untereinander eingesetzt werden. Das günstigste Mischungsverhältnis kann leicht durch Vorversuche ermittelt werden und hängt u.a. von dem zu stabilisierenden Polymeren ab.

Phenolische Antioxidantien im Sinne der Erfindung sind Verbindungen, die mindestens eine sterisch gehinderte phenolische Gruppierung enthalten.

Beispiele für solche Verbindungen sind:
2,6-Di-t-butyl-4-methylphenol, 2,4,6-Tri-t-butylphenol, 2,2'-Methylen-bis-(4-methyl-6-t-butylphenol), 2,2'-Thio-bis-(4-methyl-6-t-butylphenol), 4,4'-Thio-bis-(3-methyl-6-t-butylphenol), 4,4'-Butyliden-bis-(6-t-butyl-3-methylphenol), 4,4'-Methyliden-bis-(2,6-di-t-butylphenol), 2,2'-Methylen-bis-[4-methyl-6-(1-methylcyclohexyl)-phenol], Tetrakis[methylen-3-(3,5-di-t-butyl-4-hydroxyphenyl)-propionat]methan, 1,3,5-Trimethyl-2,4,6-tris-(3,5-di-t-butyl-4-hydroxybenzyl)benzol,N,N'-Hexamethylenbis(3,5-di-t-butyl-4-hydroxy-hydrozimtsäureamid)-,Octadecyl-3-(3,5-di-t-butyl-4-hydroxyphenyl)propionat, 1,3,5-Tris-(3,5-di-t-butyl-4-hydroxybenzyl)-isocyanurat, 1,1,3-Tris-(5-t-butyl-4-hydroxy-2-methylphenyl)butan, 1,3,5-Tris-(3,5-di-t-butyl-4-hydroxybenzyl)-mesitylen, Ethylenglykol-bis[3,3-bis(3'-t-butyl-4'-hydroxyphenyl)butyrat], 2,2'-Thiodiethyl-bis-3-(3,5-di-t-butyl-4-hydroxyphenyl)propionat,Di-(3-t-butyl-4'-hydroxy-5-methylphenyl)dicyclopentadien, 2,2'-Methylen-bis-(4-methyl-6-cyclohexylphenol), 1,6-Hexandiol-bis-(3,5-di-t-butyl-4-hydroxyphenyl)propionat, 2,4-Bis-(n-octylthio)-6-(4-hydroxy-3,5-di-t-butylanilino)-1,3,5-triazin, 3,5-Di-t-butyl-4-hydroxybenzyl-phosphonsäurediethylester und Triethylenglykol-bis-3-(t-butyl-4-hydroxy-5-methylphenyl)-propionat.

Bevorzugt sind 2,6-Di-t-butyl-4-methylphenol, Octadecyl-3-(3,5-di-t-butyl-4-hydroxyphenyl)propionat, 2,2'-Methylen-bis-(4-methyl-6-cyclohexylphenol), 2,2'-Methylen-bis-(4-methyl-6-t-butylphenol), Triethylenglykol-bis-3-(t-butyl-4-hydroxy-5-methylphenyl)-propionatund Tetrakis[methylen-3-(3,5-di-t-butyl-4-hydroxyphenyl)-propionat]methan.

Die erfindungsgemäßen Mischungen können außerdem phosphorhaltige Stabilisatoren, wie Distearylpentaerythritdiphosphit, Tris-(nonylphenyl)-phosphit, Tetrakis-(2,4-di-t-butylphenyl-4,4'-biphenylendiphosphonit, Tris-(2,4-di-t-butylphenyl)phosphit, Neopentylglykoltriethylenglykoldiphosphit, Diisodecylpentaerythritdiphosphit, Tristearylphosphit, Trilaurylphosphit, Triphenylphosphit, und/oder stickstoffhaltige Stabilisatoren, Vorzugsweise aromatische Amine, wie 4,4'-Di-t-octyldiphenylamin, 4,4'-Di-($\alpha$,$\alpha$-dimethylbenzyl)-diphenylamin, Phenyl-$\beta$-naphthylamin, N-Isopropyl-N'-phenyl-p-phenylendiamin, N-Phenyl-2-naphthylamin und/oder Phenyl-2-aminonaphthalin enthalten.

Die erfindungsgemäßen Mischungen können die genannten phosphorhaltigen und/oder stickstoffhaltigen Stabilisatoren in Mengen von bis zu 50 Gew.-Teilen, bevorzugt bis zu 45 Gew.-Teilen und besonders bevorzugt bis zu 40 Gew.-Teilen enthalten, bezogen auf 100 Gew.-Teile Mischung aus A + B.

Die erfindungsgemäßen synergistisch wirkenden Mischungen eignen sich als Mittel zur Stabilisierung von Polymeren gegen oxidativen Abbau bzw. sonstige durch Sauerstoffeinwirkung hervorgerufene Veränderungen.

Polymere, die durch Zusatz der erfindungsgemäßen Mischungen stabilisiert werden können sind z.B. Acrylnitril/Butadien/Styrol-Terpolymerisate (ABS), Methylmethacrylat/Butadien/Styrol-Terpolymerisate (MBS), Acrylnitril/Styrol/Acrylatkautschuk-Terpolymerisate (ASA), Acrylnitril/EPDM-Kautschuk/Styrol-Terpolymerisate (AES), schlagzähes Polystyrol (HIPS), Polycarbonat/ABS-Gemische, Polycarbonat/ASA-Gemische, Polycarbonat/MBS-Gemische, Polyphenylenoxid/HIPS-Gemische, kautschukmodifizierte Polyamide (Polyamid-6, Polyamid-66, Polyamid-12), kautschukmodifizierte Polyester (Polyethylenterephthalat, Polybutylenterephthalat), kautschukmodifizierte Polyolefine (Polyethylen, Polypropylen).

Prinzipiell sind die erfindungsgemäßen Mischungen auch zur Stabilisierung von Kautschuken wie z.B. Polybutadien, Polyisopren, Butadien/Styrol-Copolymeren, Butadien/Acrylnitril-Copolymeren, EPDM-Kautschuken, Polychloropren geeignet, weiterhin auch für Polyolefine wie z.B. Polyethylen und Polypropylen.

Besonders gute Wirksamkeit zeigen die erfindungsgemäßen Mischungen bei der Stabilisierung von ABS-Polymerisaten, MBS-Polymerisaten, Styrol/Acrylnitril-Copolymerisaten, $\alpha$-Methylstyrol/Acrylnitril-Copolymerisaten, schlagzähem Polystyrol, Polyolefinen und Kautschukpolymeren sowie diese Polymere enthaltenden Polymerblends, wobei neben einer sehr guten Wirksamkeit aufgrund der flüssigen bzw. leicht schmelzbaren Form eine äußerst einfache Einarbeitbarkeit gegeben ist.

Die zur Stabilisierung verwendeten Mengen der erfindungsgemäßen Mischungen betragen 0,1 bis 5 Gew.-Teile, bevorzugt 0,2 bis 4 Gew.-Teile und besonders bevorzugt 0,5 bis 3 Gew.-Teile pro 100 Gew.-Teile zu stabilisierendes Polymer.

Dabei kann die Stabilisatorzusammensetzung in fester Form, als Lösung oder in Form einer Dispersion oder einer Emulsion zu dem Polymermaterial zugegeben werden. Es ist auch möglich, die Bestandteile der Mischung in verschiedenen Formen dem Polymeren zuzusetzen. Je nach Zustand des zu stabilisierenden Polymeren arbeitet man auf Knetern, Walzen in Emulsion oder in Lösung.

Ein weiterer Gegenstand der Erfindung sind stabilisierte polymere Formmassen, die dadurch gekennzeichnet sind, daS sie 0,1 bis 5 Gew.-Teile, bevorzugt 0,2 bis 4 Gew.-Teile, besonders bevorzugt 0,5 bis 3 Gew.-Teile pro 100 Gew.-Teile Polymer der erfindungsgemäßen Mischung aus phenolischem Antioxidans und einem Thioenolether enthalten.

Beispiele

Beispiel 1 (Herstellung von Thioenolethern)

3-Cyclohexen-(1)-ylidenmethyl-n-dodecylthioether:
Ein Gemisch aus 110,1 g (1 Mol) Tetrahydrobenzaldehyd, 202,4 g (1 Mol) n-Dodecylmercaptan und 500 ml Toluol, das 2,6 g p-Toluolsulfonsäure enthält, wird 3 h am Wasserabscheider zum Sieden erhitzt. Nach beendigter Wasserabspaltung wird das Reaktionsgemisch säurefrei gewaschen und anschließend im Hochvakuum fraktioniert. Man erhält 240 g 3-Cyclohexen-(1)-ylidenmethyl-n-dodecylthioether. - Auch die in der Tabelle 1 aufgeführten Thioenolverbindungen wurden in der oben beschriebenen Weise hergestellt.

Tabelle 1

| Thioenolether | Ausbeute % d.Th. | Siedepunkt / 0,1-0,2 mb °C | Elementaranalyse berechnet % C | H | S | gefunden % C | H | S |
|---|---|---|---|---|---|---|---|---|
| 3-Cyclohexen-(1)-ylidenmethyl-n-dodecylthioether | 82 | 180-185 | 77,5 | 11,6 | 10,9 | 76,7 | 11,3 | 11,8 |
| 3-Cyclohexen-(1)-ylidenmethyl-tert.dodecylthioether | 76 | 140-145 | 77,5 | 11,6 | 10,9 | 76,1 | 11,0 | 12,0 |
| 3-Cyclohexen-(1)-ylidenmethyl-octadecylthioether | 92 | - | 79,4 | 12,2 | 8,5 | 78,5 | 12,2 | 8,9 |
| 3-Cyclohexen-(1)-ylidenmethyl-p-tolylthioether | 73 | 110-115 | 77,8 | 7,4 | 14,8 | 75,8 | 7,3 | 14,7 |
| 1-Hexenyl-n-dodecylthioether | 95 | 125-130 | 76,0 | 12,7 | 11,3 | 74,7 | 12,3 | 11,8 |
| Cyclohexylidenmethyl-n-dodecylthioether | 83 | 150-155 | 77,0 | 12,2 | 10,8 | 76,6 | 11,7 | 10,7 |
| 3-Methyl-(3-)cyclohexen-(1)-ylidenmethyl-n-dodecylthioether | 53 | 170-175 | 77,9 | 11,7 | 10,4 | 77,9 | 11,0 | 10,6 |
| 2-Methyl-1-propenyl-tert.-dodecylthioether | 63 | 100-105 | 75,0 | 12,5 | 12,5 | 71,9 | 12,0 | 13,0 |
| 3-Cyclohexen-(1)-ylidenmethyl-cyclohexylthioether | 87 | 110-115 | 75,0 | 9,6 | 15,4 | 74,2 | 9,6 | 15,3 |
| 3-Cyclohexen-(1)-ylidenmethyl-benzylthioether | 68 | 117-128 | 77,1 | 8,2 | 14,7 | 77,8 | 7,4 | 14,7 |
| 3-Cyclohexen-(1)-ylidenmethyl-n-octylthioether | 87 | 120-128 | 75,0 | 11,7 | 13,3 | 76,2 | 10,7 | 14,0 |

Beispiel 2

Die angegebenen Teile sind Gewichtsteile und beziehen sich immer auf Festbestandteile.

Alle DSC-Messungen wurden unter Verwendung eines DSC-2-Meßgerätes der Firma Perkin Elmer

durchgeführt (Spülgas Sauerstoff 3,6 1/h, Heizrate bei dynamischer Messung 20 K/min, isotherme Messung bei 180 ° C). Dazu wurde trockenes, stabilisiertes Pfropfkautschukpulver mit einer Korngröße von 0,5 bis 0,1 mm eingesetzt.

Bei der Durchführung der nachstehend beschriebenen Versuche wurden folgende Substanzen eingesetzt:

A. In Latexform vorliegender Pfropfkautschuk, erhalten durch radikalische Emulsionspolymerisation von 50 Gew.-Teilen eines Styrol/Acrylnitril = 72:28-Gemisches in Gegenwart von 50 Gew.-Teilen eines in Latexform vorliegenden Polybutadiens mit einem mittleren Teilchendurchmesser ($d_{50}$) von 0,4 $\mu$m.

B. Octadecyl-3-(3,5-di-tert.-butyl-4-hydroxyphenyl)propionat (Irganox 1076, Ciba Geigy)

C. Dilaurylthiodipropionat (Irganox PS 800, Ciba Geigy)

D. 3-Cyclohexen-(1)-ylidenmethyl-n-dodecylthioether

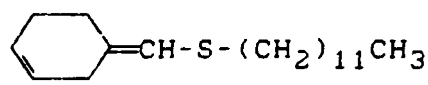

E. 1-Hexenyl-n-dodecylthioether

$CH_3-(CH_2)_3-CH=CH-S-(CH_2)_{11}CH_3$

F. Cyclohexylidenmethyldodecylthioether

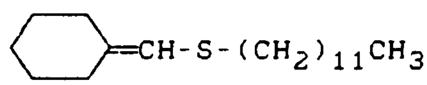

G. 3-Methyl-(3)-cyclohexen-(1)-ylidenmethyl-n-dodecylthioether

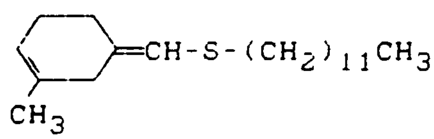

H. 3-Cyclohexen-(1)-ylidenmethyl-octadecylthioether

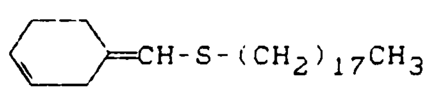

I. 3-Cyclohexen-(1)-ylidenmethyl-tert.-dodecylthioether

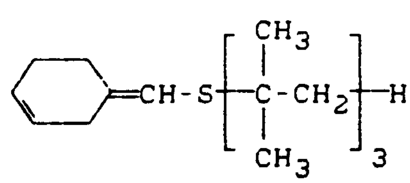

K. 2-Methyl-1-propenyl-tert.dodecylthioether

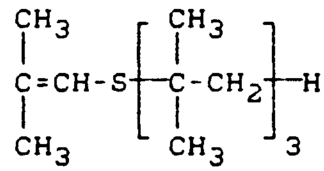

Herstellung einer Stabilisatorendispersion unter Verwendung des phenolischen Antioxidans B und einer schwefelhaltigen Komponente (C bis K)

Zu einer Mischung von 50 Gew.-Teilen phenolischem Antioxidans B und 50 Gew.-Teilen schwefelhaltiger Komponente wird unter starkem Rühren (Ultraturrax) eine heiße Lösung von 6,5 Gew.-Teilen eines nichtionischen Emulgators (ethoxyliertes Nonylphenol) in 93,5 Gew.-Teilen Wasser gegeben und bis zum Vorliegen einer stabilen Dispersion gerührt. Die bis zum Vorliegen der stabilen Dispersionen notwendigen Rührzeiten sind in Tabelle 2 angegeben.

Stabilisierung des Pfropfkautschuklatex A und Aufarbeitung

Dem unter A beschriebenen Pfropfkautschuklatex wurden so viel der oben beschriebenen Stabilisatordispersionen zugefügt, daß pro 100 Gew.-Teile Pfropfkautschuk ein Gehalt von 1 Gew.-Teil phenolischem Antioxidans B und 1 Gew.-Teil schwefelhaltiger Komponente resultierte. Dann wurde der Latex mit einer Lösung eines Magnesiumsulfat/Essigsäuregemisches koaguliert, nach Filtration des Fällgemisches das Polymerisat abgetrennt und bei 70 °C im Vakuum getrocknet. Das Pfropfkautschukpulver wurde mit einer Prüfsiebmaschine mit Vibrationssieben (Hersteller Haver und Boecker) gesiebt, wonach die Siebfraktion mit einer Korngröße im Bereich von 0,5 bis 0,1 mm für die DSC-Untersuchungen eingesetzt wurden.

Prüfung der Eigenfarbe von ABS-Polymerisaten nach thermoplastischer Verarbeitung

40 Gew.-Teile des nicht gesiebten Pfropfkautschukpulvers wurden mit 60 Gew.-Teilen eines Styrol/Acrylnitril = 72:28-Copolymerisats und 2 Gew.-Teilen eines Gleitmittels (Pentaerythrittetrastearat) auf einem Innenkneter homogen vermischt, wonach die Mischung granuliert und durch Spritzgießen bei 240 °C zu ebenen Platten verarbeitet wurde.

An diesen Platten erfolgte die visuelle Beurteilung der Eigenfarbe nach folgender Skala:

1 = sehr hell

2 = hell

3 = leichte Gelbverfärbung

Tabelle 2

| Beispiel | Eingesetzte schwefelhaltige Verbindung | Rührzeit bis zum Vorliegen einer stabilen Dispersion | Ergebnisse der DSC-Messungen Isotherm (Peak) | Dynamisch (Peak) | Beurteilung der Eigenfarbe |
|---|---|---|---|---|---|
| 1 | D | 70 sec | 107 min | 237° C | 1 |
| 2 | E | 50 sec | 59 min | 230° C | 1 |
| 3 | F | 60 sec | 46 min | 229° C | 1 |
| 4 | G | 50 sec | 43 min | 227° C | 1 |
| 5 | H | 50 sec | 41 min | 228° C | 1 |
| 6 | I | 40 sec | 38 min | 226° C | 2 |
| 7 | K | 60 sec | 35 min | 228° C | 2-3 |
| 8 (Vergleich) | C | 90 sec | 49 min | 229° C | 2 |

## Patentansprüche

**1.** Mischung aus

A) 1 bis 99 Gew.-Teilen eines Thioenolethers der Formel

$R^1=CH-S-R^2$ ,

worin

$R^1$ einen geradkettigen oder verzweigten Alkenylrest mit 3 bis 18 C-Atomen oder einen substituierten oder unsubstituierten Cycloalkenylrest mit 5 bis 8 C-Atomen bedeutet und

$R^2$ für einen geradkettigen oder verzweigten Alkylrest mit 3 bis 18 C-Atomen, einen Arylrest mit 6 bis 10 C-Atomen oder einen Aralkylrest mit 7 bis 12 C-Atomen steht, wobei der Alkylrest noch zusätzlich in der Kette durch ein Heteroatom oder mehrere Heteroatome unterbrochen sein kann,

und

B) 99 bis 1 Gew.-Teilen eines phenolischen Antioxidans.

**2.** Verwendung der Mischungen gemäß Anspruch 1 zur Stabilisierung von Polymeren.

**3.** Polymere Formmassen enthaltend eine Mischung gemäß Anspruch 1 in Mengen von 0,1 bis 5 Gew.-%, bezogen auf das Polymere.

**4.** Polymere Formmassen gemäß Anspruch 3 enthaltend ABS-Polymerisate, MBS-Polymerisate, schlagzähes Polystyrol, Polyolefin oder Kautschukpolymere.